# EUROPÄISCHE PATENTSCHRIFT

(11) **EP 0 631 508 B1**
(45) Veröffentlichungstag und Bekanntmachung des Hinweises auf die Patenterteilung: **04.06.1997**
(21) Anmeldenummer: 93906567.8
(22) Anmeldetag: 19.03.1993
(51) Int. Cl.: A61M 1/00, A61M 25/00, A61M 16/04

(54) **ABSAUGKATHETER**
SUCTION CATHETER
CATHETER D'ASPIRATION

(30) Priorität: 20.03.1992 DE 4208912
(43) Veröffentlichungstag der Anmeldung: 04.01.1995
(60) Teilanmeldung: 96101316.6
(73) Patentinhaber: Schön, Rudolf, D-53127 Bonn (DE)
(72) Erfinder: Schön, Rudolf, D-53127 Bonn (DE)
(74) Vertreter: Müller-Gerbes, Margot, Dipl.-Ing.
(86) Internationale Anmeldenummer: EP9300672
(87) Internationale Veröffentlichungsnummer: WO9318801

(56) Entgegenhaltungen:
- US-A- 2 504 557
- US-A- 2 574 135
- US-A- 3 848 604
- US-A- 3 945 385
- Internationale Norm (ISO):Masstabelle für Trachealtuben

## Beschreibung

Die Erfindung betrifft einen Absaugkatheter gemäß dem Oberbegriff des Anspruches 1.

Die Erfindung betrifft des weiteren eine Weiterbildung des Absaugkatheters, der am proximalen Ende mit einem Anschlußstück oder dergleichen verbindbar ist und wobei das Lumen am distalen Ende des Katheterrohres eine gegenüber dem Querschnitt des Lumens vergrößerte distale Austrittsöffnung des Lumens aufweist.

Absaugkatheter der gattungsgemäßen Art sind beispielswiese aus der DE-AS 2364119 bzw. US-PS 3848 604 bekannt.

Aus der US-PS 2130 406 ist ein Saugschnorchel zum Absaugen von Speichel aus dem Mundraum für zahnärztliche Zwecke bekannt, der an seinem distalen Ende außenseitig verdickt ist, wobei die Verdickung durch radial verlaufende Kerben außenseitig unterbrochen ist.

Einen ähnlich gestalteten Saugschnorchel für zahnärztliche Zwecke zeigt die US-PS 2574 135. Hierbei ist das Schnorchelende in Gestalt eines zylindrischen Kolbens verdickt und weist außenseitige, bis an die distale Austrittsöffnung reichende Kerben auf.

Beim Absaugen von Schleim aus dem Tracheobronchialraum mittels Absaugkathetern kann ein andauerndes Vakuum während des Absaugens zu ernsten Verletzungen der Schleimhaut führen. Zum einen besteht die Gefahr, daß der Absaugkatheter an der Schleimheit klebt und direkt von ihr fortgezogen wird. Absaugkatheter sind üblicherweise zusätzlich zu der distalen Austrittsöffnung am Ende mit mindestens einem Seitenloch ausgestattet. Wenn der Absaugkatheter in enge Berührung mit der Schleimhaut kommt, hebt er durch den Unterdruck die Schleimhaut an und invaginiert sie in diese Seitenlöcher oder auch in die Austrittsöffnung. Die dadurch entstehenden Verletzungen dienen als Brutstätte für Bakterien und führen zu Ödemen usw.

Bei dem aus der DE-A-2364119, die zur Bildung des Oberbegriffs des Anspruchs 1 herangezogen worden ist, bekannten Katheter ist zur Vermeidung von Verletzungen der Schleimhaut am distalen Ende des Absaugkatheters ein abgerundeter Ringwulst ausgebildet, der radial am Ende des Katheters über den Umfang des Katheters vorsteht. Mit Hilfe dieses Ringwulstes wird der Katheter innerhalb der Luftröhre zentriert und soll des weiteren ein laminares Luftpolster schaffen, damit er nicht mit der Wand der Luftröhre in Berührung kommt und auch keine Schleimhaut in den Katheter eingesogen werden soll. Es hat sich jedoch herausgestellt, daß nicht in allen Fällen das Einziehen von Schleimhaut in die radialen seitlichen Öffnungen des Katheters vermieden werden kann. Darüber hinaus stellt sich in manchen Fällen das Problem, daß die distale Austrittsöffnung durch Schleim verstopft wird. Die Austrittsöffnung dieses Katheters kann durch Abrundung leicht gegenüber dem Querschnitt des Lumens vergrößert ausgebildet sein.

Der Erfindung liegt die Aufgabe zugrunde, die bekannten Absaugkatheter konstruktiv zu verbessern, so daß ein Verstopfen des distalen Endes durch Schleim beim Absaugen vermieden wird und auch das Festkleben oder Einsaugen von Schleimhaut in die distale Austrittsöffnung bzw. die seitlichen Öffnungen im Bereich des distalen Katheterendes mit Sicherheit vermieden werden.

Zur Lösung dieser Aufgabe wird vorgeschlagen, den gattungsgemäßen Absaugkatheter gemäß den kennzeichnenden Merkmalen des Anspruches 1 auszubilden. Eine vorteilhafte Weiterbildung wird für einen Absaugkatheter mit den Merkmalen des Anspruches 4 vorgeschlagen, bei dem das Lumen zum distalen Ende des Katheterrohres des Absaugkatheters hin in Form eines Trichters erweitert ist.

Durch Ausbildung des distalen Endbereiches des Absaugkatheters als zylindrischer Kolben wird eine flächige Führung des Absaugkatheters in den Luftwegen, wie Luftröhre, geschaffen, wodurch auch verhindert wird, daß die Schleimhäute in die in Richtung auf das proximale Ende des Absaugkatheters folgenden Belüftungsöffnungen des Katheters angesaugt werden. Es wird auch das Verstopfen der Belüftungsöffnungen durch Schleim vermieden.

Auf diese Weise wird eine Schädigung von Submuscosa und Muscosa auch in diesem Bereich vermieden. Bei allen bekannten Absaugkathetern, die keine Ringwulst am distalen Ende aufweisen, treten immer wieder Schäden an den Schleimhäuten im Bereich der Belüftungsöffnungen des Absaugkatheters auf. Auch die Absaugkatheter mit Ringwulst am distalen Ende verhindern dann ein Ansaugen der Schleimhäute im Bereich der radialen Belüftungsöffnungen nicht, wenn das distale Austrittsende durch Schleim verstopft.

Die vorliegende Erfindung schafft hier Abhilfe, da sie durch spezielle Ausgestaltung im Bereich der Austrittsöffnung ein Zusetzen derselben mit zähem Sekret verhindert, d.h. ein zügiges Absaugen ermöglicht und damit ein Festsaugen des Absaugkatheters an den Schleimhäuten über die Belüftungsöffnungen verhindert. Es hat sich herausgestellt, daß über die radialen Belüftungsöffnungen Luft nachgezogen wird, so daß beim Absaugen von Sekreten aus dem Bronchialraum die Lungensegmente nicht kollabieren. Um diese Ventilation zu ermöglichen, ist das Einhalten bestimmter Abstände zwischen den Belüftungsöffnungen und den im distalen Endbereich des Absaugkatheters ausgebildeten verdickten Bereichen in Form eines zylindrischen Kolbenteiles von Bedeutung, um diese Ventilation zu erhalten. Dieser Abstand zwischen den Belüftungsöffnungen des Absaugkatheters und dem proximalen Beginn der zylindrischen Außenwand des Kolbens sollte kleiner als 6 mm sein. Dieser Abstand ist unabhängig von der Größe des Absaugkatheters einzuhalten.

Vorteilhafte Ausgestaltungen von Absaugkathetern der gattungsgemäßen Art sind den kennzeichnenden Merkmalen der Unteransprüche entnehmbar.

Neben der trichterförmigen Erweiterung des Austrittes des Lumens am distalen Ende des Absaugkatheters ist es auch möglich, den Trichter und/oder die Lumenwandungen mittels in die Wandungen eingedrückter in axialer Richtung verlaufende Kerben zu erweitern. Bei Erweiterung der distalen Austrittsöffnung des Lumens sowohl mittels einer trichterförmigen Erweiterung als auch mittels Kerben können die Kerben gleichweit, weniger weit oder weiter als die trichterförmige Erweiterung in axialer Richtung sich in das Lumen hinein erstrecken.

Die vorteilhafte Erweiterung der distalen Austrittsöffnung in Verbindung mit dem als Dichtungs- und Führungskolben dienenden verdickten Bereich nahe dem distalen Ende des Absaugkatheters vermeidet sicher ein Verstopfen des distalen Endes des Absaugkatheters. Hierzu trägt auch die erfindungsgemäße Ausbildung des Katheterrohres am distalen Ende bei, die, im axialen Querschnitt betrachtet, verjüngt mittels der Übergangszone endet.

Überraschend hat sich herausgestellt, daß auch bei sehr zähem Sekret, das mit Hilfe des Absaugkatheters aus dem Bronchialraum abgesaugt werden soll, bei Ansetzen des Unterdruckes das Sekret die distale Austrittsöffnung des Absaugkatheters nicht verstopft, sondern zügig abgesaugt wird. Zugleich wird auch das Festsaugen des Absaugkatheters bzw. des distalen Endbereiches an den Schleimhäuten vermieden. Jede, auch die geringste Invagination der Schleimhäute wird vermieden. Dies wird darauf zurückgeführt, daß infolge der trichterförmigen Erweiterung des Lumens zur distalen Austrittsöffnung hin ein verbessertes Ansaugen des Schleimes und Ableitung in das Lumen erfolgt.

Die zusätzlichen Kerben in Teilbereichen der distalen Austrittsöffnung, die in die Wandungen der trichterförmigen Erweiterung eingedrückt sind, sind relativ klein ausgeführt, jedoch über den erweiterten Austrittsbereich bis in den normalen Lumenbereich hin ausgebildet. Bei einer bevorzugten Ausgestaltung reichen die Kerben gleichweit, vom distalen Ende des Katheterrohres her betrachtet, wie ein gleichfalls eingeformter Trichter in das Lumen in axialer Richtung hinein.

Eine wesentliche Ausgestaltung des erfindungsgemäßen Absaugkatheters ist auch darin zu sehen, daß der im distalen Endbereich des Absaugkatheters ausgebildete verdickte Bereich in Gestalt des zylindrischen Kolbens mit seinem Außendurchmesser die Größe in CH (Charr) nach Iso Norm des Absaugkatheters bestimmt und das hieran bis zum proximalen Ende des Absaugkatheters anschließende Katheterrohr mit dem gegenüber, d.h. gegenüber dem zylindrischen Kolben und dem distalen Endbereich, verkleinerten Außendurchmesser ausgebildet ist. Bisher wird üblicherweise die Größe des Absaugkatheters in Charriere nach dem Außendurchmesser des langen Katheterrohres bestimmt, beispielsweise auch bei Absaugkathetern nach der DE-AS 2364119.

Erfindungsgemäß wird also zur Festlegung der Größen des Absaugkatheters, gemessen in CH nach Iso Norm, der Außendurchmesser des zylindrischen Kolbens des verdickten Bereiches innerhalb des distalen Endbereiches in Verbindung mit dem Durchmesser des durchgängigen, zum Absaugen benutzten Lumens herangezogen.

Die erfindungsgemäße Ausbildung des distalen Endbereiches mit einem zylindrischem Kolben, der mit seinem Außendurchmesser über das sonstige Katheterrohr vorsteht, kann auch bei Absaugkathetern mit zwei Lumen, wie sie beispielsweise in der DE-PS 3608943 beschrieben sind, angewendet werden.

Bevorzugte Ausgestaltungen eines Absaugkatheters mit einem durch einen verdickten Bereich in Gestalt eines zylindrischen Kolbens innerhalb des distalen Endbereiches des Katheterrohres ausgebildeten Absaugkatheters, der die Führung des Absaugkatheters beim Einführen in das Trachealbronchialsystem eines Patienten übernimmt, sind den Unteransprüchen entnehmbar.

Für die einwandfreie Funktion des erfindungsgemäßen Absaugkatheters als atraumatischer Katheter ist die Größe des Kolbens und vorteilhafterweise die Größe des Trichters der trichterartigen Erweiterung der distalen Austrittsöffnung ausschlaggebend.

Zur Verhinderung der Verstopfung der distalen Austrittsöffnung des Absaugkatheters kann die vorteilhafte Erweiterung des Lumens am Austrittsende des Katheterrohres als Trichter, kegelförmig oder trompetenförmig, aufgeweitet oder mittels in die Lumenwandungen eingedrückter bis zum distalen Ende des Katheterrohres reichende Kerben oder einer Kombination von trichterartiger Erweiterung und Kerben ausgebildet sein.

Die bevorzugten Abmessungen für die Ausgestaltung des Absaugkatheters gemäß der Erfindung nach den Merkmalen der Ansprüche 6 bis 10 sind auf die genormten Absaugkathetergrößen von 8 CH bis 18 CH ausgerichtet, wobei übliche Fertigungstoleranzen zu berücksichtigen sind. Das Katheterrohr für die Absaugkatheter wird aus geeigneten sterilisierbaren thermoplastischen Kunststoffen, die extrudierbar sind, durch Extrusion mit nachfolgender Verformung hergestellt.

Der Absaugkatheter bzw. das Katheterrohr sind aus einem nicht toxischen flexiblen biegsamen Material, wie beispielsweise natürlichem oder synthetischem Gummi, Polypropylen, Polyethylen, Polyvinylchlorid oder Polyamiden, hergestellt.

Hierbei kann die Steifigkeit des Materials so gewählt werden, daß auch bei langen Absaugkathetern, die in der Regel bei den angegebenen Größen von 8 CH bis 18 CH eine Länge zwischen 50 bis 65 cm aufweisen, eine ausreichende Steifigkeit und Knicksicherheit erreicht wird, auch bei sehr geringen Wandstärken des Katheterrohres.

Die Länge a des zylindrischen Kolbens sollte ausreichend sein, um je nach Kathetergröße eine ausreichende flächige Anlage und Führung des Katheters bzw. Katheterendes in den Luftwegen zu ermöglichen. Die sich an beiden Seiten anschließenden Übergangszonen bilden konische oder annähernd konische oder abgerundete Übergänge von dem zylindrischen Kolben auf der einen Seite unmittelbar in eine axiale Endwulst des Katheterrohres an der distalen Austrittsöffnung und auf der anderen Seite zum sich zum proximalen Ende hin erstreckenden Katheterrohr. Diese Übergänge sind flach geneigt und bevorzugt an beiden Seiten unter gleichen Winkeln und etwa gleichlang auszubilden.

Durch das Zusammenwirken des am distalen Ende des Lumens vorteilhafterweise ausgebildeten Trichters und die damit gegenüber dem Lumenquerschnitt im Querschnitt vergrößerte Austrittsöffnung einerseits und der außenseitigen Übergangszone vom Kolben zum distalen Ende des Katheterrohres mit konischer Verjüngung oder abgerundeter Verjüngung oder abgerundeter Verjüngung des Querschnittes des Katheterrohres andererseits mündet das distale Ende des Katheterrohres sich verjüngend in einem abgerundeten Ende, das einen kleineren Außendurchmesser als der Kolben des verdickten Bereiches des Katheterrohres aufweist. Dieses Ende ermöglicht eine leichte sanfte Einführung des Absaugkatheters in die Luftröhre eines Patienten.

Das proximale Ende des Absaugkatheters kann mit einem Anschlußstück, beispielsweise Trichter, versehen sein, jedoch auch mit anderen Teilen ausgerüstet oder an Geräte angeschlossen werden. Das proximale Ende des Absaugkatheters kann selbst auch konisch erweitert ausgebildet werden.

Der distale Endbereich des Absaugkatheters ist üblicherweise in gerader Längserstreckung des Katheterrohres vorgesehen, er kann jedoch auch in bezug auf die Längsachse des Absaugkatheters ein- oder mehrfach abgebogen ausgebildet sein. Bei derartigen Absaugkathetern ist der verdickte Bereich zur Führung des Absaugkatheters innerhalb des distalen Endbereiches vor dem abgebogenen Endteil, d.h. im geraden Teil des Katheterrohres, auszubilden.

Die Erfindung wird in der Zeichnung anhand eines Ausführungsbeispieies näher erläutert. Es zeigen
- Figur 1: in schematischer Darstellung im Längsschnitt nicht maßstabgerecht einen Absaugkatheter
- Figur 2: die Aufsicht auf das distale Ende des Absaugkatheters nach Figur 1 in vergrößerter Darstellung
- Figur 3: den Schnitt CC nach Figur 2 für den distalen Endbereich des Absaugkatheters
- Figur 3a: die Figur 3 mit zusätzlichen Angaben für die Abmessungen des Absaugkatheters
- Figur 4: eine Aufsicht auf eine weitere Ausgestaltung des distalen Endbereiches des Absaugkatheters
- Figur 5: den Schnitt AA nach Figur 4
- Figur 6: den Schnitt BB nach Figur 4 für den distalen Endbereich.

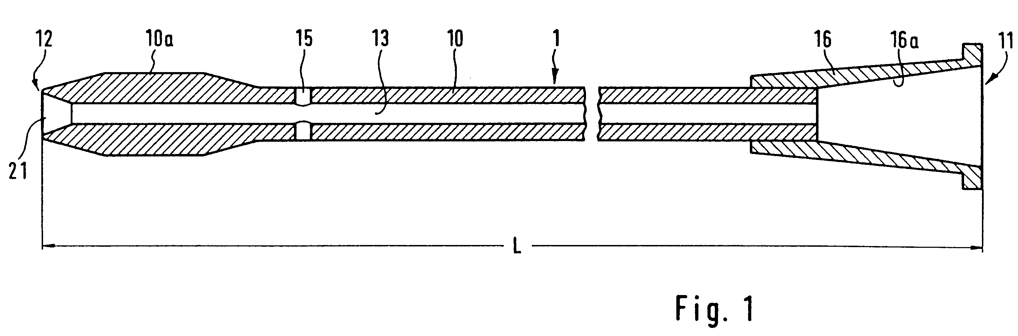

In der Figur 1 ist schematisch ein Absaugkatheter 1 zum Absaugen von Sekreten und anderen Fluiden aus dem Tracheobronchialraum eines Patienten dargestellt, der durch die Luftröhre des Patienten bis in den Bronchialraum einführbar ist. Der Absaugkatheter ist an seinem proximalen Ende 11 in dem gezeigten Beispiel mit einem Anschlußstück 16, beispielsweise Trichter mit Innentrichter 16a fest verbunden. Der Absaugkatheter kann auch mit anderen Anschlußstücken verbunden werden. Der Absaugkatheter 1 enthält das Katheterrohr 10, das innerhalb distalen Endbereiches mit einer Verdickung in Gestalt eines zylindrischen Kolbens 10a ausgebildet ist. Das einzige durchgängige in dem Katheterrohr ausgebildete Lumen 13 des Absaugkatheters 1 mündet am distalen Ende 12 in der distalen Austrittsöffnung 21. Absaugkatheter der dargestellten Art weisen eine Länge L üblicherweise zwischen etwa 50 bis 65 cm auf. Der Innendurchmesser des durchgängigen Lumens 13 beträgt beispielsweise bei einer Kathetergröße CH 10 1,54 mm und bei einer Kathetergröße CH 16 3,0 mm. Der Absaugkatheter 1 nach Figur 1 weist erfindungsgemäß im distalen Endbereich eine kolbenförmige Verdickung gegenüber dem übrigen Katheterrohr 10 auf, und zwar in Gestalt des zylindrischen Kolbens 10a. Der für die Normgrößenbestimmung des Absaugkatheters 1 zugrundezulegende Außendurchmesser ist erfindungsgemäß derjenige des Kolbens 10a als dem dicksten Teil des in den Trachealbronchialraum einzuführenden Bereiches des Katheterrohres. Der Außendurchmesser des an dem verdickten Bereich 10a, 17, 18 anschließenden Katheterrohres 10, das sich zum proximalen Ende 11 des Absaugkatheters erstreckt, ist gegenüber dem Außendurchmesser 10a des Kolbens kleiner bemessen, d.h. das Katheterrohr 10 ist gegenüber dem Kolben 10a kleiner.

An den Kolben 10a des Absaugkatheters schließen sich in Richtung auf das proximale Ende 11 des Absaugkatheters relativ nahe Belüftungsöffnungen 15 an, die sich radial durch die Wandung des Katheterrohres 10 erstrecken und die das Lumen 13 mit der Umgebung verbinden. Hierbei sind die Belüftungsöffnungen 15 als Bohrungen gleichmäßig über den Umfang des Katheterrohres 10 verteilt angeordnet, beispielsweise zwei oder vier Belüftungsöffnungen 15.

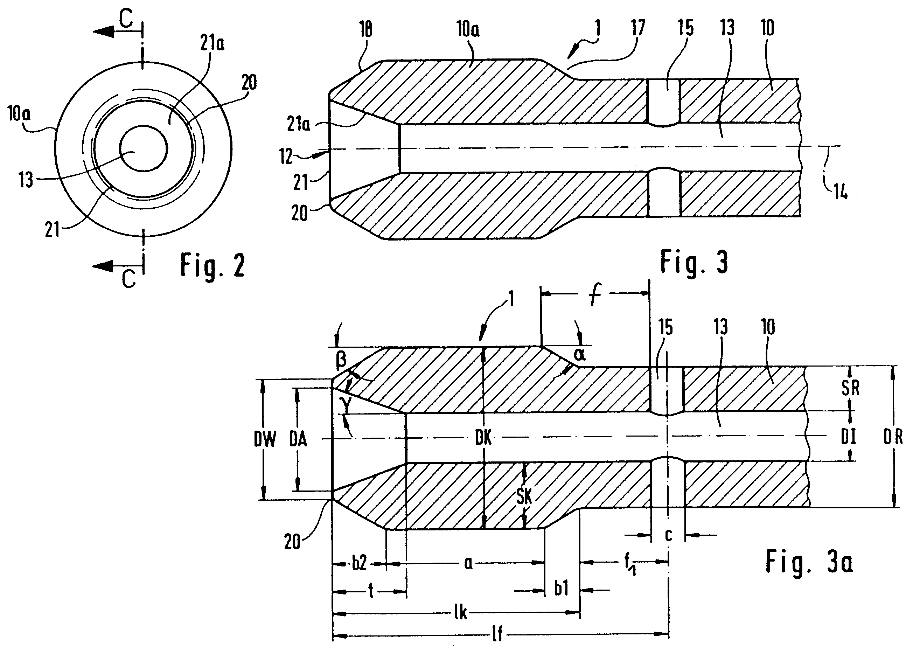

In den Figuren 2 bis 3a ist der distale Endbereich des Absaugkatheters 1 nach Figur 1 vergrößert dargestellt und hieran werden die erfindungsgemäßen Ausgestaltungen näher erläutert.

Aus dem Längsschnitt durch den distalen Endbereich des Absaugkatheters 1 nach Figur 3 wird die keulenförmige Ausgestaltung des distalen Endbereiches mit dem zylindrischen Kolben 10a deutlich. Der zylindrische Kolben 10a fällt nach beiden Seiten, d.h. sowohl zum distalen Ende 12 als auch zum proximalen Ende hin über eine konische Übergangszone 18 bzw. 17 ab. Im distalen Endbereich 12 geht die Übergangszone 18 unmittelbar in das abgerundete Ende 20, das die distale Austrittsöffnung 21 begrenzt, über. Zum proximalen Ende hin geht die Übergangszone 17 in das Katheterrohr 10, d.h. dessen Außenwandung, über. Alle Übergänge sind fließend und abgerundet ausgebildet.

Das durchgängige Lumen 13, das in der Längsachse 14 des Absaugkatheters 1 koaxial verläuft, ist zum distalen Ende 12 hin trichterförmig erweitert. Diese trichterförmige Erweiterung 21a mündet in der Austrittsöffnung 21. Die Austrittsöffnung 21 des durchgängigen Lumens 13 am distalen Ende 12 des Absaugkatheters 1 ist damit größer im Querschnitt als der Querschnitt des Lumens 13. Die trichterförmige Erweiterung des Lumens des Katheterrohres ist von geraden oder leicht gerundeten Wänden begrenzt. Die Austrittsöffnung 21 wird von dem abgerundeten Ende 20 des Katheterrohres begrenzt, der das abgerundete axiale Ende des Katheterrohres bildet. Dieser ringförmige axiale Wulst 20 geht außenseitig über eine konische Übergangszone 18 unmittelbar in den zylindrischen Kolben 10a über. Im Querschnitt betrachtet, ist der Wulst konisch beidseitig verjüngt ausgebildet.

In proximaler Richtung schließt sich an den zylindrischen Kolben 10a des distalen Endbereiches nach der konischen Übergangszone 17 unter Verjüngung das Katheterrohr 10 an, und unmittelbar danach sind auch die Belüftungsöffnungen 15, die das Lumen 13 mit der Umgebung verbinden, in den Wandungen des Katheterrohres ausgebildet. Wenn nun der Absaugkatheter 1 in ein Bronchialsystem zum Absaugen eingeführt ist und Vakuum angelegt wird, wird Sekret in Richtung auf die distale Austrittsöffnung 21 aus dem Bronchialraum angesaugt. Der an die Austrittsöffnung 21 vorteilhafterweise anschließende ende Trichter verhindert das Zusetzen der Austrittsöffnung 21 und des Lumens 13 mit zähem Sekret, da infolge der trichterförmigen Erweiterung neben dem Sekret noch ein Luftstrom mit angesaugt wird, so daß das zähe Sekret in das Lumen 13 eingesaugt und damit abgeführt werden kann. Die trichterförmige Erweiterung des distalen Endes des Lumens 13 verhindert in Verbindung mit der außenseitigen Führung des Katheters mittels des Kolbens 10a das Verstopfen bzw. Zusetzen des distalen Endes des Lumens mit Sekret. Im Bereich der Belüftungsöffnungen wird zusätzlich eine Ventilation ermöglicht, so daß hier ebenfalls im Seitenbereich insbesondere auch durch den Freiraum zwischen dem Kolben 10a und dem Katheterrohr 10 ein Ansaugen von Schleimhäuten, d.h. ein Festkleben derselben und damit Beschädigung derselben verhindert wird.

Auch die Ausbildung des sich verjüngenden Endes 20 des Katheterrohres an der Austrittsöffnung 21 verhindert das Festsetzen durch Sekret.

Für eine gute Funktion des erfindungsgemäß mit einem kolbenförmig verdicktem distalen Endbereich ausgebildeten Absaugkatheters sind die Abmessungen des Kolbens und zusätzlich der trichterförmigen Erweiterung von Bedeutung. Hierzu sind gemäß Figur 3a die bevorzugten Maße angegeben. Die Winkel α und β für die etwa konischen Übergänge des zylindrischen Kolbens 10a in Richtung distales Ende 12 und in Richtung proximales Ende, d.h. zum Katheterrohr 10, betragen zwischen 10° bis 20° und sollten abgerundete Übergänge aufweisen. Der Innendurchmesser DI für Kathetergrößen 8 bis 18 CH liegt zwischen 1,0 bis 4,0 mm bei einem Außendurchmesser DK gemessen im Bereich des zylindrischen Kolbens am distalen Endbereich des Absaugkatheters zwischen 2,7 und 6,1 mm zuzüglich Fertigungstoleranzen. Hieraus ergeben sich Wandstärken SK im Bereich des zylindrischen Kolbens von etwa 0,7 bis 1,5 mm. Der gegenüber dem Kolben 10a verjüngte Außendurchmesser des Katheterrohres 10 DR beträgt etwa zwischen 1,7 bis 5,1 mm, so daß sich hieraus Wandstärken SR für das Katheterrohr von etwa 0,3 bis 0,9 mm ergeben. Der Durchmesser c der Belüftungsöffnungen beträgt zwischen 0,25 bis 0,8 mm. Die Tiefe t der trichterförmigen Erweiterung sollte zwischen 2 bis 4 mm betragen, der Durchmesser DA der Austrittsöffnung am Ausgang, d.h. im Bereich des Endwulstes 20 etwa 1,25 bis 4,3 mm betragen, d.h. etwa 0,2 bis 0,7 mm größer als der jeweils zugehörige Innendurchmesser des Lumens 13 sein. Der Außendurchmesser DW des Endwulstes 20 sollte zwischen 0,4 bis 0,8 mm größer als der Durchmesser DA der Austrittsöffnung sein. Hierbei sind stets die kleineren Werte den kleineren CH-Größen des Absaugkatheters zuzuordnen.

Die Länge b1, b2 der Übergangszonen 17, 18 des distalen Endbereiches, d.h. von dem Kolben 10a zu den Bereichen sollte vorzugsweise gleich lang gewählt sein, wobei diese Längen zwischen etwa 1,5 mm bis 3,0 mm betragen.

Die Länge a des zylindrischen Kolbens 10a sollte mindestens 2 mm betragen bis maximal 8 mm und vorzugsweise im Bereich von etwa 3,0 bis 5 mm liegen. Wesentlich für eine ausreichende Ventilation durch die Belüftungsöffnungen 15 bei eingeführtem Absaugkatheter in das Bronchialsystem ist der Freiraum zwischen dem Kolben 10a und den Belüftungsöffnungen 15, d.h. der Abstand f bzw. f1. Der Abstand f1 wird vorzugsweise mit konstanter Größe für alle Katheter gewählt, vorzugsweise etwa 2,0 bis 3,0 mm. Die Gesamtlänge Lf des erfindungsgemäß gestalteten distalen Endbereiches vom distalen Ende bis zur Bohrungsachse der Belüftungsöffnungen 15 sollte 6 bis 16 mm betragen und entsprechend den Kathetergrößen von CH 8 bis CH 18 ebenfalls vorzugsweise konstant sein und jeweils zwischen 8 bis 12 mm, vorzugsweise 10 mm betragen. Die Länge lk des gegenüber dem Katheterrohr 10 verdickten Bereiches 17, 10a, 18 des Katheterrohres beträgt vorzugsweise etwa 7 bis 9 mm.

Ein Absaugkatheter CH 12 weist bei erfindungsgemäßer Ausgestaltung beispielsweise folgende Abmessungen auf (Fertigungstoleranzen sind hinzurechenbar): DI 2,0 mm, DR 3,0 mm, DK 4,0 mm, DA 2,31 mm, DW 3,0 mm, t 3,25 mm bis 4,0 mm, b1, b2 1,65 mm, lk 7,5 mm; f1 2,5 mm; lf 10 mm, α/β 17°; c 0,8 mm.

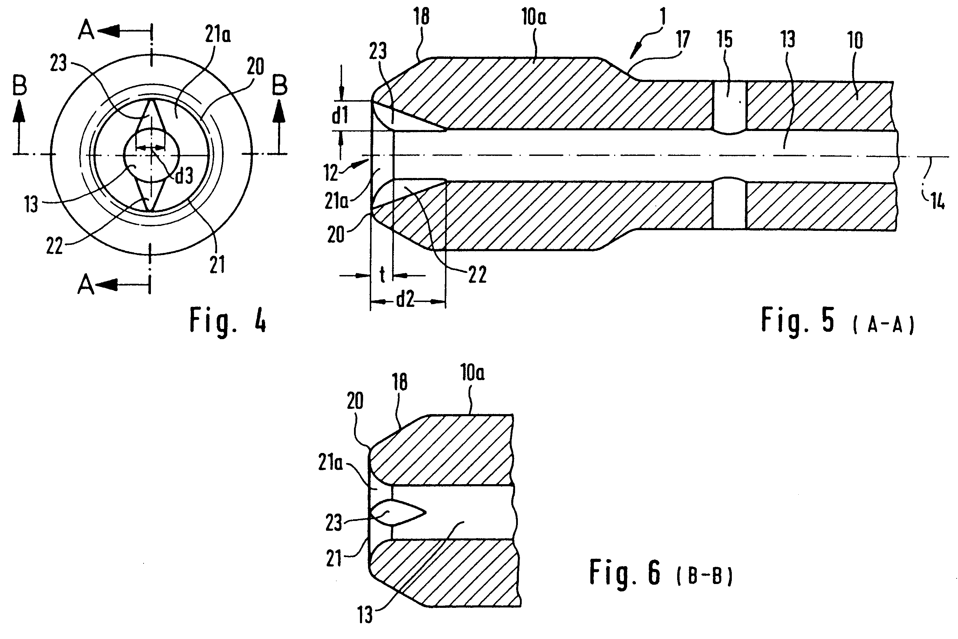

In den Figuren 4 bis 6 ist ein erfindungsgemäßer Absaugkatheter 1 dargestellt, dessen distaler Endbereich eine kleine trichterförmige Erweiterung 21a sowie zusätzliche in die den Trichter 21a und das Lumen 13 begrenzende Wandung eingedrückte axial verlaufende Kerben 21, 22 aufweist.

Die distale Austrittsöffnung 21 bildet eine Kreisfläche, deren Querschnitt größer als der Querschnitt des Lumens 13 ist. Diese distale Austrittsöffnung 21 ist durch zusätzliche Kerben 22, 23 in Teilbereichen radial erweitert. Diese Kerben 22, 23, die gleichmäßig über den Umfang verteilt sind, beispielsweise zwei einander gegenüberliegende Kerben, wie dargestellt, oder zusätzlich zwei weitere hierzu um 90° versetzte Kerben, vertiefen den Trichter der Austrittsöffnung an zwei Stellen in radialer Richtung. Diese Kerben 22, 23 verlaufen jedoch nicht nur im Bereich des Trichters der Austrittsöffnung 21, sondern erstrecken sich weiter über den Katheter in axialer Richtung bis hinein in das Lumen 13. Dies ist auch aus der Ansicht nach Figur 4 ersichtlich.

Die im Bereich des distalen Austrittes des Lumens 13 ausgebildeten Kerben sollten eine Tiefe d1, gemessen von der Lumenwandung in radialer Richtung von d1, von etwa 0,1 bis 0,3 mm aufweisen und eine Länge d2, gemessen vorn distalen Ende in axialer Richtung, von etwa 2 bis 4 mm. Die Breite d3 der Kerben, siehe Figur 2, sollte in jedem Fall kleiner als der Durchmesser des Lumens 13 sein, insbesondere etwa ein 1/7 bis 1/4 des Durchmessers DI des Lumens 13 betragen.

Die übrige Gestaltung und Abmessungen des Absaugkatheters gemäß Figur 4 bis 6 entsprechen denen des Absaugkatheters gemäß Figur 2 bis 3a.

Mit dem erfindungsgemäß ausgebildeten Absaugkatheter, d.h. der Ausbildung des distalen Endbereiches werden jegliche Verletzungen beim Absaugen der Schleimhäute und auch ein Verstopfen des Absaugkatheters mit Sekreten vermieden.

Es ist auch möglich, auf eine Erweiterung des Austrittsendes des Lumens eines Absaugkatheters gemäß Figur 1 gegebenenfalls zu verzichten, jedoch die Führung des Katheterrohres innerhalb des Trachealbronchialraumes eines Patienten durch die Ausbildung eines zylindrisch verdickten kolbenförmigen Bereiches 10a nahe am distalen Ende des Katheterrohres zu bewirken. Der kolbenförmge Bereich sollte hierbei eine Länge von etwa 2 mm bis etwa 8 mm aufweisen. Durch den sich zum distalen Ende hin verjüngenden Außenquerschnitt des Katheterrohres wird auch ein Zustopfen des Lumenendes mit Sekret vermieden. Dies könnte insbesondere bei großen Querschnitten des Lumens 13 des Katheterrohres 10 eines Absaugkatheters gemäß Figur 1 von Vorteil sein.

## Patentansprüche

1. Absaugkatheter zum Absaugen aus dem Trachealbronchialsystem eines Patientes, enthaltend ein biegsames Katheterrohr (10) mit einem proximalen Ende (11) und einem distalen Ende (12) und mindestens einem durchgehend ausgebildeten Lumen (13) sowie in einem distalen Endbereich des Katheterrohres ausgebildeten Belüftungsöffnungen (15), die in das Lumen führen, wobei dieser distale Endbereich einen verdickten Bereich, angeordnet zwischen den Belüftungsöffnungen und dem distalen Ende des Katheterrohres, einschließt, **dadurch gekennzeichnet**, daß der verdickte Bereich die Form eines Kolbens (10a) mit einer zylindrischen Außenwand und proximalen und distalen Übergangszonen (17,18), die der zentralen zylindrischen Außenwand benachbart sind, bildet, und bei Größen des Absaugkatheters von 8 CH bis 18 CH der Kolben einen Außendurchmesser DK von zwischen 2,7 und 6,1 mm und die zentrale zylindrische Außenwand eine Länge (a) zwischen etwa 2 mm bis 8 mm, insbesondere 3 bis 5 mm erhält, und die Länge (a) der zentralen zylindrishen Außenwand dazu bestimmt ist, eine flächige Führung und Anlage des Absaugkatheters mittels dieses verdickten Bereiches zu ermöglichen, wenn er in das Trachealbronchialsystem eines Patienten eingeführt wird, und die Belüftungsöffnungen (15) benachbart dem distalen Endbereich nahe der zentralen zylindrischen Außenwand in einer vorbestimmten Distanz (Lf) ausgehend von der Bohrungsachse der Belüftungsöffnungen bis zum distalen Ende (12) des Katheterrohres von etwa 6 mm bis 16 mm positioniert sind.

2. Absaugkatheter nach Anspruch 1,
**dadurch gekennzeichnet**, daß die vorbestimmte Länge (a) der zentralen zylindrischen Außenwand etwa gleich groß wie der Durchmesser (DK) des verdickten Bereiches des distalen Endbereiches ist.

3. Absaugkatheter nach Anspruch 1,
**dadurch gekennzeichnet**, daß die Länge (a) der zentralen zylindrischen Außenwand mindestens das zweifache des Durchmessers (DI) des Lumens (13) beträgt.

4. Absaugkatheter nach einem der Ansprüche 1 bis 3,
der am proximalen Ende mit einem Anschlußstück oder dergleichen verbindbar ist und wobei das Lumen am distalen Ende des Katheterrorhes eine gegenüber dem Querschnitt des Lumens vergrößerte distale Austrittsöffnung des Lumens aufweist, **dadurch gekennzeichnet**, daß das Lumen (13) zum distalen Ende des Katheterrohres (10) in Form eines Trichters (21a) erweitert ist, wobei der Trichter ausgehend vom distalen Ende (12) eine Tiefe (t) von etwa 2 bis 4 mm aufweist.

5. Absaugkatheter nach einem der Ansprüche 1 bis 4,
**dadurch gekennzeichnet**, daß zur Festlegung der Größen des Absaugkatheters, gemessen in CH (Charr) nach ISO-Norm, der Außendurchmesser (DK) des zylindrischen Kolbens (10a) des verdickten Bereiches des den Absaugkatheter bildenden Katheterrohres (10) in Verbindung mit dem Durchmesser (DI) des durchgängigen zum Absaugen benutzten Lumens (13) herangezogen ist und das hieran bis zum proximalen Ende (11) des Absaugkatheters anschließende Katheterrohr (10) mit gegenüber dem Außendurchmesser (DK) des zylindrischen Kolbens (10a) verkleinertem Außendurchmesser (DR) ausgebildet ist.

6. Absaugkatheter nach Anspruch 1 bis 3,
**dadurch gekennzeichnet**, daß die Differenz zwischen der Wanddicke (SK) des zylindrischen Kolbens (10a) und der Wanddicke (SR) des Katheterrohres (10) zwischen 0,4 bis 0,6 mm bei Kathetergrößen von 8 bis 18 CH beträgt.

7. Absaugkatheter nach einem der Ansprüche 1 bis 6,
**dadurch gekennzeichnet**, daß bei Größen des Absaugkatheters von 8 CH bis 18 CH die Wanddicke (SK) des zylindrischen Kolbens (10a) zwischen 0,7 bis 1,5 mm beträgt und die Wanddicke (SR) des Katheterrohres (10) zwischen 0,3 und 0,9 mm beträgt.

8. Absaugkatheter nach einem der Ansprüche 1 bis 7,
**dadurch gekennzeichnet**, daß zwischen dem zylindrischen Kolben (10a) einschließlich Übergangszone (17) und der Bohrungsachse der Belüftungsöffnungen (15) im distalen Endbereich des Absaugkatheters (1) ein Abstand (f1) von 2 bis 3 mm vorhanden ist.

9. Absaugkatheter nach einem der Ansprüche 1 bis 8,
**dadurch gekennzeichnet**, daß die Übergangszonen (17,18) von dem zylindrischen Kolben (10a) nach beiden Seiten unter einem flachen Winkel (α, β) von etwa 10° bis 20° verlaufen.

10. Absaugkatheter nach einem der Ansprüche 1 bis 9,
**dadurch gekennzeichnet**, daß die Gesamtlänge (Lf) des distalen Endbereiches mit verdicktem Bereich (10a, 17, 18) von den Belüftungsöffnungen (15) bis zuin distalen Ende (12) für Absaugkatheter der Größen 8 CH bis 18 CH jeweils gleich lang ausgebildet ist und im Bereich von etwa 8 bis 12 mm liegt.

11. Absaugkatheter nach einem der Ansprüche 4 bis 10,
**dadurch gekennzeichnet**, daß das Lumen zum distalen Ende hin durch mindestens zwei gleichmäßig über den Umfang verteilt angeordnete, vom distalen Ende (12) ausgehende, sich axial erstreckende, in die den Trichter (21a) und gegebenenfalls das Lumen (13) begrenzende Wandung des Katheterrohres (10) eingedrückte Kerben (22, 23) erweitert ist.

12. Absaugkatheter nach Anspruch 11,
**dadurch gekennzeichnet**, daß die Kerben (22, 23) eine axiale Länge (d2) von etwa 2 bis 4 mm aufweisen und eine Breite (d3) kleiner als der Durchmesser des Lumens, insbesondere etwa 1/7 bis 1/4 des Lumendurchmessers betragend, haben.

## Claims

1. Suction catheter for sucking from the tracheobronchial system of a patient, comprising a flexible catheter tube (10) with a proximal end (11) and a distal end (12) and at least one through lumen (13) as well as ventilation openings (15) formed in a distal end area of the catheter tube, said openings leading into the lumen, this distal end area includes a thickened area located between the ventilation openings and the distal end of the catheter tube, characeterized in that the thickened area has the shape of a piston (10a) with a cylindrical outside wall and proximal and distal transition zones (17,18) adjacent to the central cylindrical outside wall and for suction catheter sizes from 8 CH to 18 CH the outer diameter DK of the piston is between 2,7 and 6,1 mm and the central cylindrical outside wall has a predetermined length (a) between approximately 2 mm and 8 mm, especially 3 to 5 mm and with the length (a) of the central cylindrical outside wall being such as to permit areal guidance and fitting of the suction catheter by this thickened area when it is introduced into the tracheobronchial system of a patient and the ventilation openings (15) formed adjacent outside wall are positioned at a predetermined distance (Lf) starting from the axis of the ventilation openings to the distal end (12) of the catheter tube of approximately 6 to 16 mm.

2. Suction catheter according to claim 1 characterized in that the predetermined length (a) of the central cylindrical outside wall is roughly the same as diameter (DK) of the thickened area of the distal end area.

3. Suction catheter according to claim 1 characterized in that the length (a) of the central cylindrical outside wall is at least twice the diameter (DI) of the lumen (13).

4. Suction catheter according to one of the claims 1 to 3 being connectable at the proximal end with a connector or the like and whereby the lumen is provided at the distal end of the catheter tube with a distal outlet opening of the lumen being enlarged with respect to the cross section of the lumen, characterized in that the lumen (13) is expanded in the shape of a funnel (21a) toward the distal end of catheter tube (10) of the suction catheter, whereby the funnel, beginning at the distal end (12), has a depth (t) of approximately 2 to 4 mm.

5. Suction catheter according to one of the claims 1 to 4 characterized in that, in order to establish the sizes of the suction catheter, measured in CH (Charr) according to the ISO standard, the outside diameter DK of cylindrical piston (10a) of the thickened area of catheter tube (10) forming the suction catheter is used in conjunction with the diameter DI of through lumen (13) used for suction and catheter tube (10) abutting it up to proximal end (11) of the suction catheter has an outside diameter (DR) that is reduced relative to outside diameter (DK) of cylindrical piston (10a).

6. Suction catheter according to claims 1 to 3 characterized in that the difference between the wall thickness (SK) of cylindrical piston (10a) and the wall thickness (SR) of catheter tube (10) is between 0.4 and 0.6 mm at catheter sizes of 8 to 18 CH.

7. Suction catheter according to one of claims 1 to 6 characterized in that wall thickness (SK) of cylindrical piston (10a) is between 0.7 and 1,5 mm at suction catheter sizes from 8 CH to 18 CH and wall thickness (SR) of catheter tube (10) is between 0.3 and 0.9 mm.

8. Suction catheter according to one of claims 1 to 7 characterized in that a space (f1) of 2 to 3 mm is provided between cylindrical piston (10a), including transition zone (17), and the axis of the ventilation openings (15) in the distal end area of suction catheter (1).

9. Suction catheter according to one of claims 1 to 8 characterized in that transition zones (17, 18) of cylindrical piston (10a) run at a shallow angle (α, β) of about 10° to 20° to both sides.

10. Suction catheter according to one of claims 1 to 9 characterized in that the total length (Lf) of the distal end area with thickened area (10a, 17, 18) is made the same length from ventilation openings (15) to distal end (12) for suction catheters of sizes 8 CH to 18 CH and is in the range from about 8 to 12 mm.

11. Suction catheter according to one of the claims 1 to 10 characterized in that the lumen is expanded toward the distal end by at least two axially extended notches (22, 23) uniformly distributed over the circumference, said notches departing from distal end (12) and impressed into the wall of the catheter tube (10) delimiting the funnel (21a) and possibly lumen (13).

12. Suction catheter according to claim 11 characterized in that the notches (22, 23) have an axial length (d2) of approximately 2 to 4 mm and a width (d3) less than the diameter of the lumen, especially approximately 1/7 to 1/4 of the lumen diameter.

## Revendications

1. Cathéter d'aspiration pour réaliser une aspiration dans le système trachéo-bronchique d'un patient, comprenant un tuyau flexible (10) de cathéter doté d'une extrémité proximale (11) et d'une extrémité distale (12) et d'au moins une lumière (13) traversante ainsi que d'orifices de ventilation (15) pratiqués dans un segment terminal distal du tuyau du cathéter et communiquant avec l'intérieur de la lumière, ce segment terminal distal incluant une zone renflée disposée entre les orifices de ventilation et l'extrémité distale du tuyau du cathéter, caractérisé en ce que la zone renflée a la forme d'une ampoule (10a) dotée d'une paroi externe cylindrique et de zones de transition proximale et distale (17, 18) qui sont voisines de la paroi externe cylindrique centrale, et en ce que, pour des calibres du cathéter d'aspiration de l'ordre de 8 CH à 18 CH, l'ampoule reçoive un diamètre extérieur (DK) de l'ordre de 2,7 à 6,1 mm et la paroi externe cylindrique centrale, une longueur (a) de l'ordre d'environ 2 mm à 8 mm, en particulier de 3 à 5 mm, et en ce que la longueur (a) de la paroi externe cylindrique centrale soit définie de sorte que le cathéter d'aspiration puisse être guidé et agencé selon la surface au moyen de cette zone renflée quand il est introduit dans le système trachéo-bronchique d'un patient, et en ce que les orifices de ventilation (15) sont positionnés au voisinage du segment terminal distal, tout près de la paroi externe cylindrique centrale, à une distance prédéterminée (Lf), mesurée de l'axe d'alésage des orifices de ventilation jusqu'à l'extrémité distale (12) du tuyau du cathéter, de l'ordre de 6 à 16 mm.

2. Cathéter d'aspiration selon la revendication 1, caractérisé en ce que la longueur prédéterminée (a) de la paroi externe cylindrique centrale est à peu près identique au diamètre (DK) de la zone renflée du segment terminal distal.

3. Cathéter d'aspiration selon la revendication 1, caractérisé en ce que la longueur (a) de la paroi externe cylindrique centrale fait au moins le double du diamètre (DI) de la lumière (13).

4. Cathéter d'aspiration selon l'une quelconque des revendications 1 à 3, qui peut être connecté par son extrémité proximale à une pièce de raccordement ou l'équivalent et dans lequel la lumière, à l'extrémité distale du tuyau du cathéter, présente un orifice distal de sortie agrandi par rapport à la section transversale de la lumière, caractérisé en ce que la lumière (13), en direction de l'extrémité distale du tuyau (10) du cathéter, est élargie en forme d'entonnoir (21a), lequel présente une profondeur (t), mesurée à partir de l'extrémité distale (12), de l'ordre d'environ 2 à 4 mm.

5. Cathéter d'aspiration selon l'une quelconque des revendications 1 à 4, caractérisé en ce que, pour déterminer les calibres du cathéter d'aspiration, mesurés en CH (Charr) selon la norme ISO, on se sert du diamètre extérieur (DK) de l'ampoule cylindrique (10a) de la zone renflée du tuyau (10) constituant le cathéter d'aspiration en relation avec le diamètre (DI) de la lumière (13) traversante utilisée pour aspirer, et en ce que le tuyau (10) qui y fait suite jusqu'à l'extrémité proximale (11) du cathéter d'aspiration est doté d'un diamètre extérieur (DR) plus petit que le diamètre extérieur (DK) de l'ampoule cylindrique (10a).

6. Cathéter d'aspiration selon les revendications 1 à 3, caractérisé en ce que la différence entre l'épaisseur (SK) de la paroi de l'ampoule cylindrique (10a) et l'épaisseur (SR) de la paroi du tuyau (10) du cathéter est comprise entre 0,4 et 0,6 mm pour des calibres de cathéter de 8 à 18 CH.

7. Cathéter d'aspiration selon l'une quelconque des revendications 1 à 6, caractérisé en ce que, pour des cathéters d'aspiration d'un calibre allant de 8 CH à 18 CH, l'épaisseur (SK) de la paroi de l'ampoule cylindrique (10a) est de l'ordre de 0,7 à 1,5 mm et l'épaisseur (SR) de la paroi du tuyau (10) du cathéter, de l'ordre de 0,3 à 0,9 mm.

8. Cathéter d'aspiration selon l'une quelconque des revendications 1 à 7, caractérisé en ce que, entre l'ampoule cylindrique (10a), y compris la zone de transition (17), et l'axe d'alésage des orifices de ventilation (15) pratiqués dans le segment terminal distal du cathéter d'aspiration (1), il y a un écart (f1) de 2 à 3 mm.

9. Cathéter d'aspiration selon l'une quelconque des revendications 1 à 8, caractérisé en ce que les zones de transition (17, 18) de l'ampoule cylindrique (10a) s'étendent, des deux côtés, sous un angle plan (α, β) d'environ 10° à 20°.

10. Cathéter d'aspiration selon l'une quelconque des revendications 1 à 9, caractérisé en ce que la longueur totale (Lf) du segment terminal distal doté de la zone renflée (10a, 17, 18), mesurée des orifices de ventilation (15) à l'extrémité distale (12), est toujours la même et est de l'ordre d'environ 8 à 12 mm, et ce pour des cathéters d'aspiration dont le calibre va de 8 CH à 18 CH.

11. Cathéter d'aspiration selon l'une quelconque des revendications 4 à 10, caractérisé en ce que la lumière, en direction de l'extrémité distale, est élargie par au moins deux entailles (22, 23) qui sont distribuées uniformément sur la circonférence, prennent naissance à l'extrémité distale (12), s'étendent axialement et sont pratiquées dans la paroi du tuyau (10) du cathéter limitant l'entonnoir (21a) et facultativement, la lumière (13).

12. Cathéter d'aspiration selon la revendication 11, caractérisé en ce que les entailles (22, 23) ont une longueur axiale (d2) d'environ 2 à 4 mm et une largeur (d3) inférieure au diamètre de la lumière, en particulier de l'ordre d'environ 1/7 à 1/4 du diamètre de la lumière.
